# FASCICULE DE BREVET EUROPEEN

(11) **EP 0 638 986 B1**
(45) Date de publication et mention de la délivrance du brevet: **18.11.1998**
(21) Numéro de dépôt: 94401757.3
(22) Date de dépôt: 01.08.1994
(51) Int. Cl.: H02M 3/335

(54) **Convertisseur continu-continu pour charge à tension variable**
Gleichstrom-Gleichstromumformer für eine Last mit veränderlicher Spannung
DC-DC converter for a variable voltage load

(30) Priorité: 10.08.1993 FR 9309807
(43) Date de publication de la demande: 15.02.1995
(73) Titulaire: ELA MEDICAL (Société anonyme), F-92541 Montrouge Cédex (FR)
(72) Inventeur: Jacobson, Peter, F-67500 Haguenau (FR)
(74) Mandataire: Laget, Jean-Loup

(56) Documents cités:
- EP-A- 0 042 043
- US-A- 4 037 271
- US-A- 4 548 209
- PATENT ABSTRACTS OF JAPAN vol. 15, no. 98 (E-1042) 8 Mars 1991 & JP-A-02 307 361 (ELCO CO LTD) 20 Décembre 1990

## Description

L'invention concerne des circuits convertisseurs continu-continu, et plus particulièrement des circuits convertisseurs fonctionnant avec une tension de charge variable, tels que des circuits de charge de condensateurs. Un exemple est constitué par la charge d'un condensateur de stockage de chocs à haute énergie dans un défibrillateur implantable, à partir d'une pile.

Ces circuits à tension de charge variable stockent habituellement l'énergie dans un inducteur, pour adapter la tension d'entrée à la tension de sortie avec seulement des pertes parasites. Dans ce cas, l'inducteur est un inducteur à enroulement unique, ou à enroulements multiples avec une borne commune (autotransformateur), ou à enroulements multiples sans borne commune (transformateur de découplage).

L'invention concerne également la prévention d'interférences entre un convertisseur continu-continu et un système de communication électromagnétique.

Dans le cas présent, le terme défibrillateur est utilisé pour désigner tout dispositif prévu pour arrêter une tachyarythmie avec une énergie électrique qui dépasse sensiblement l'énergie fournie par des stimulateurs cardiaques implantables. Ceci inclut les défibrillateurs/appareils de cardioversion/stimulateurs cardiaques implantables et les défibrillateurs/stimulateurs cardiaques implantables.

Le condensateur de stockage à haute énergie d'un défibrillateur peut consister en un condensateur, ou en plusieurs condensateurs reliés en série, en parallèle, ou les deux.

Les défibrillateurs implantables fournissent généralement un choc tronqué par décharge d'un condensateur avec une tension maximale approximativement de 0,75 kV. Ces dispositifs utilisent des piles à basse tension, de l'ordre de 3 à 15 V, pour constituer leur alimentation de puissance primaire. Ils doivent également comprendre un circuit pour convertir l'énergie de la pile en énergie qui charge le condensateur à haute tension. Ce circuit doit fonctionner avec un rendement élevé, pour préserver la durée de vie de la pile. Il doit également charger le ou les condensateurs aussi rapidement que possible pour permettre de délivrer une thérapie de défibrillation aussi tôt que possible quand la condition du patient le demande.

Les circuits de charge de condensateurs de l'art antérieur utilisent fréquemment un convertisseur élévateur, du type dénommé flyback, du fait de son efficacité et de sa simplicité. Un convertisseur flyback typique fonctionne avec un cycle en deux parties et comprend un inducteur. Pendant la première partie du cycle, appelée phase d'entrée, le courant augmente dans l'inducteur, stockant l'énergie dans son champ magnétique. Pendant la seconde partie du cycle, appelée phase de sortie, le courant diminue dans l'inducteur, transférant l'énergie à la charge. Une diode série empêche l'énergie de s'écouler dans la charge pendant la phase d'entrée.

Au début de la phase d'entrée, un circuit de commande ferme un commutateur qui applique la tension de la source d'entrée à un enroulement de l'inducteur. La tension aux bornes de l'enroulement amène le courant à monter dans l'enroulement, stockant l'énergie dans un champ magnétique de l'inducteur. La pente de la rampe augmente en proportion directe de la tension de la source d'entrée.

A la fin de la phase d'entrée, le circuit de commande ouvre le commutateur entre la source d'entrée et l'enroulement de l'inducteur. L'énergie stockée dans le champ amène le courant à continuer à circuler par tout trajet disponible, comprenant un enroulement de l'inducteur. Le circuit flyback n'offre qu'un trajet comprenant la diode série et la charge, et ainsi l'inducteur transfère l'énergie à la charge. Le courant diminue jusqu'à ce qu'aucune énergie ne subsiste dans l'inducteur ou jusqu'à ce que le contrôleur ferme le commutateur d'entrée et fasse remonter le courant. La pente de la rampe pendant la phase de sortie augmente en proportion directe de la tension de charge, par exemple en proportion directe de la tension du condensateur de stockage d'énergie que charge le convertisseur.

Ainsi, la durée nécessaire pour transférer une quantité donnée d'énergie de la source d'entrée à l'inducteur pendant la phase d'entrée dépend inversement de la tension de la source d'entrée, et la durée nécessaire au transfert de cette énergie de l'inducteur à la charge pendant la phase de sortie dépend inversement de la tension de charge.

Dans l'art antérieur:

Les brevets USRE 27652 et 27757 au nom de Mirowski décrivent un moyen de défibrillation automatique, avec un convertisseur continu-continu, une source d'entrée consistant en une pile d'approximativement 15 V, et une charge de sortie consistant en un condensateur chargé entre 0 et 2,5 KV. Mirowski valide le convertisseur en fermant un relais qui relie la source d'entrée au convertisseur, mais ne donne pas de détails concernant le convertisseur lui-même.

Les brevets U S No 4 316 472 au nom de Mirowski, 4 827 936 au nom de Pless et 4 823 796 au nom de Benson montrent un convertisseur comprenant un moyen pour autoriser et interdire l'application d'énergie au convertisseur de façon sélective quand la tension appliquée au condensateur de stockage atteint une valeur prédéterminée ou préprogrammée.

Le brevet US No 4 614 192 au nom de Imran montre un circuit de charge dans lequel les émissions radio-fréquences provenant d'un transformateur de charge transmettent l'information de charge à un démodulateur et à un décodeur externes pour obtenir une lecture des performances du dispositif implanté destinée à un médecin.

Le brevet US No 4 548 209 au nom de Wielders montre un convertisseur flyback fonctionnant à une fréquence fixe et à phase d'entrée de durée variable. Le circuit de commande fait démarrer la phase d'entrée sur chaque front d'un signal d'horloge de fréquence fixe, et il met fin à la phase d'entrée soit lorsque le courant primaire dépasse une valeur préétablie qui est au-dessous de la saturation de l'inducteur, soit à un moment fixe, avant le front suivant du signal d'horloge, soit après une seconde durée fixe plus courte s'il constate que la tension de la pile est au-dessous d'une valeur préétablie.

Le brevet US No 4 800 883 au nom de Winstrom montre un convertisseur flyback fonctionnant avec une durée de phase d'entrée fixe et une fréquence variable. Le circuit de commande fait démarrer la phase d'entrée soit après une durée de phase de sortie maximale fixe, soit lorsque la tension de l'inducteur tombe pendant la phase de sortie, indiquant qu'il ne reste plus d'énergie dans l'inducteur.

La publication EP-A-42043 décrit un convertisseur flyback.

Il est donc clair qu'un convertisseur qui charge le condensateur de stockage d'énergie dans un défibrillateur implantable a besoin d'un circuit de commande pour autoriser la charge afin d'atteindre et de maintenir une tension cible prédéterminée. Un contrôleur d'un convertisseur flyback a en outre besoin de définir les durées de la phase d'entrée et de la phase de sortie, pour éviter une saturation de l'inducteur, pour éviter de tirer trop de courant de la pile et faire ainsi baisser la tension de la pile, et pour effectuer un ajustement de la durée décroissante dont il a besoin pour transférer une quantité donnée d'énergie de l'inducteur au condensateur de stockage lorsque ce condensateur est en charge. Le convertisseur ne doit pas non plus interférer avec un système de communication électromagnétique situé au voisinage, entre l'implant et un dispositif externe de programmation et de lecture. L'art antérieur vise ces objectifs, mais avec des insuffisances:
1. Les circuits des brevets USRE 27652 et 27757 au nom de Mirowski ont besoin d'un relais électromécanique volumineux et potentiellement non fiable pour autoriser sélectivement la charge du condensateur. En outre, ces brevets n'enseignent pas la façon dont le convertisseur est mis en oeuvre.
2. Le circuit du brevet US No 4 614 192 au nom de Imran n'achemine qu'un signal binaire de communication d'un bit (le chargeur fonctionne ou non) vers un dispositif externe de lecture. Dans des dispositifs implantables plus modernes, un système séparé à couplage électromagnétique communique avec un dispositif externe de programmation et de lecture. Les perfectionnements par rapport à Imran comprennent la bidirectionnalité et la capacité d'acheminer une variété d'informations numériques et analogiques stockées ou en temps réel en ce qui concerne l'implant. Cependant, l'utilisation d'une inductance dans un convertisseur d'un implant avec un système de communication électromagnétique doit traiter le problème de l'interférence entre le convertisseur et le système.
3. Les circuits des brevets US No 4 316 472 au nom de Mirowski, 4 827 936 au nom de Pless, et 4 823 796 au nom de Benson utilisent un commutateur à semiconducteur pour autoriser sélectivement la charge du condensateur, mais n'enseignent pas la façon dont le convertisseur doit être mis en oeuvre. En outre, leurs circuits pour autoriser la charge ne montrent pas de moyens assurant que le chargeur fonctionne pendant au moins une durée minimale chaque fois que le circuit indique qu'il a besoin d'être chargé, et s'arrête pendant une certaine durée minimale chaque fois que le circuit indique que la charge est terminée. En fait, aucun circuit de l'art antérieur qui règle le fonctionnement du convertisseur en régime intermittent ne limite la fréquence des périodes d'activité. Quand le condensateur de stockage atteint sa charge totale, le chargeur doit "réapprovisionner" le condensateur en fonctionnement de temps en temps pour remplacer l'énergie perdue par des fuites ou dans les circuits de mesure de tension. Ces circuits de charge fonctionnent à plusieurs ampères, avec des fronts de montée allant jusqu'à une centaine d'ampères par microseconde, et ils produisent ainsi des interférences couplées magnétiquement à leur fréquence de fonctionnement fondamentale, et également à la fréquence des périodes d'activité. Pour réduire les interférences dues au couplage magnétique, notamment avec un système de communication électromagnétique, les périodes d'activité doivent avoir lieu à une fréquence qui est sensiblement plus basse que la fréquence du système.
4. Le circuit flyback du brevet US No 4 548 209 au nom de Wielders, fonctionnant à une fréquence fixe avec une durée variable pour la phase d'entrée, semble ne pas être approprié pour une application avec une tension de charge secondaire variable. Au début de charge, lorsqu'il faut beaucoup de temps pour transférer l'énergie stockée de l'inducteur au condensateur, Wielders indique que le circuit commence la phase d'entrée alors que de l'énergie est toujours présente dans l'inducteur. Dans ce cas, le courant de l'inducteur part d'une valeur différente de zéro pendant la phase d'entrée, et Wielders doit raccourcir la phase d'entrée pour maintenir la même énergie de pic stockée et éviter la saturation de l'inducteur. La commande du commutateur d'entrée lorsque le courant passe dans l'inducteur augmente les pertes dans ce commutateur. Inversement, à la fin de charge, lorsqu'il faut peu de temps pour transférer l'énergie stockée de l'inducteur au condensateur, le champ magnétique peut tomber à zéro avant que le contrôleur fasse redémarrer la phase d'entrée. Ce "temps mort" pendant lequel aucun courant ne passe s'ajoute inutilement à la durée totale nécessaire à la charge du condensateur de stockage d'énergie.
5. Le circuit du brevet US No 4 800 883 au nom de Winstrom, qui fonctionne à durée de phase d'entrée fixe et à fréquence variable, semble plus approprié que le circuit de Wielders pour charger un condensateur. Cependant, Winstrom définit la fin de la phase de sortie par la mesure du changement de tension de l'inducteur qui résulte de la disparition du champ magnétique quand l'inducteur a totalement transféré son énergie au condensateur de stockage. L'amplitude de ce signal varie directement avec la tension de charge. En outre, ce signal comprend des composantes de courant alternatif haute fréquence dues à la résonance de l'inductance de l'enroulement secondaire et de l'inductance de fuite primaire avec la capacitance parasite secondaire ( particulièrement prononcée quand on utilise des enroulements secondaires bifilaires comme le recommande Winstrom).

Aussi bien le circuit Wielders (qui mesure le courant de l'inducteur pour définir la fin de la phase d'entrée) que le circuit Winstrom (qui mesure la tension de l'inducteur pour définir la fin de la phase de sortie) reposent sur la mesure de signaux à fronts de montée très rapides. Amener ces signaux sur un circuit intégré utilisé de préférence pour mettre en oeuvre le contrôleur chargeur dans un dispositif implantable revient en fait à créer des problèmes de couplage magnétique et capacitif. En outre, Winstrom doit traiter un signal de déclenchement qui varie en amplitude directement à mesure que le condensateur de stockage se charge, et qui comprend des composantes de résonance, ce qui nécessite un circuit additionnel pour extraire une information de temps valable.

En conséquence, le but de l'invention telle que définie par les caractéristiques de la revendication 1 est d'améliorer la conception du convertisseur continu-continu pour charges à tension variable, le perfectionnement consistant à:
1. Faire fonctionner un convertisseur flyback avec une durée de phase d'entrée fixe, et ajuster la durée de la phase de sortie pour qu'elle corresponde à la durée nécessaire au transfert vers la charge de toute l'énergie stockée dans l'inducteur, sans exiger la mesure de signaux qui changent rapidement.
2. Quand il est utilisé à proximité d'un système de communication électromagnétique, régler la fréquence fondamentale minimale du convertisseur de façon significative au-dessus de la fréquence de fonctionnement de ce système.
3. Quand il est utilisé de façon intermittente à proximité d'un système de communication électromagnétique, autoriser et interdire sélectivement le fonctionnement du convertisseur à une fréquence maximale qui est de façon significative au-dessous de la fréquence de fonctionnement de ce système.

Dans le mode de réalisation préféré, l'invention prévoit un contrôleur pour commander le commutateur du courant d'entrée dans un convertisseur flyback. Le contrôleur détermine une durée de phase d'entrée fixe (durée de fermeture du commutateur) et une durée de phase de sortie variable (durée d'ouverture du commutateur). Lorsque le convertisseur fonctionne de façon intermittente, le contrôleur fonctionne pendant une durée minimale, et cesse de fonctionner pendant une durée minimale, ce qui correspond à une fréquence de façon significative au-dessous de la fréquence de fonctionnement du système de télémétrie.

Le contrôleur définit une durée de phase de sortie maximale pour maintenir la fréquence de fonctionnement en flyback de façon significative au-dessus de la fréquence du système de télémétrie. Quand cela est possible sans franchir cette limite de fréquence minimale, il définit la durée de la phase de sortie à une valeur qui est proportionnelle à la tension moyenne de source d'entrée et inversement proportionnelle à la tension de charge de sortie. Ceci met fin à la phase de sortie juste au moment où l'énergie stockée dans l'inducteur tombe à zéro, car la quantité de flux magnétique stockée pendant la phase d'entrée varie en proportion directe de la tension de la source d'entrée, et la durée nécessaire pour réduire ce flux jusqu'à zéro varie en proportion directe du flux de pic et en proportion inverse de la tension de charge. La tension moyenne de la source d'entrée et la tension de charge de sortie changent très lentement, sur plusieurs cycles du convertisseur, ce qui permet de réaliser des circuits de mesure simples.

L'invention a pour objet un convertisseur continu-continu, comprenant:
- un moyen inductif pour stocker l'énergie,
- un moyen de commutation pour autoriser sélectivement le courant d'entrée de l'inducteur,
- un moyen à diode pour autoriser le courant de sortie de l'inducteur seulement quand le courant d'entrée de l'inducteur est sensiblement nul, et
- un moyen de contrôle pour faire effectuer de façon répétée des cycles audit moyen de commutation pour qu'il se ferme pendant une première durée, puis qu'il s'ouvre pendant une seconde durée, caractérisé en ce qu'il comprend un moyen de compensation de sortie pour ajuster ladite seconde durée en proportion inverse de la tension de charge de sortie.

Selon d'autres caractéristiques :

La tension de charge de sortie est la tension moyenne de charge de sortie.

La tension de charge de sortie est la tension de pic de charge de sortie.

Ledit moyen de contrôle comprend :
- un premier moyen monostable pour déterminer ladite première durée,
- un second moyen monostable pour déterminer ladite seconde durée, comprenant ledit moyen de compensation de sortie,
- des moyens logiques pour faire fonctionner de façon alternée et répétée lesdits premier et second moyens monostables pour former un oscillateur astable dont la période est essentiellement égale à la somme desdites première et seconde durées.

Ledit second moyen monostable comprend :
- un condensateur pour déterminer une durée,
- un moyen pour remettre à l'état initial ledit condensateur à une première tension prédéterminée lorsque lesdits moyens logiques ne font pas fonctionner ledit second moyen monostable,
- un moyen à seuil pour déterminer le moment où la tension aux bornes dudit condensateur atteint une tension de seuil prédéterminée,
- un moyen de compensation de sortie pour charger ou décharger ledit condensateur vers ladite tension de seuil à un courant essentiellement proportionnel à la tension de charge de sortie,
- un moyen pour indiquer la fin dudit second intervalle auxdits moyens logiques, activé par ledit moyen à seuil.

Ledit moyen pour charger ou décharger comprend une résistance branchée entre la tension de charge de sortie et la borne dudit condensateur reliée audit moyen à seuil.

Un moyen de compensation d'entrée additionnel est prévu pour ajuster ladite seconde durée en proportion directe de la tension de source d'entrée.

Ladite tension de source d'entrée est la tension moyenne de source d'entrée.

Ladite tension de source d'entrée est la tension de pic de source d'entrée.

Ledit moyen de détermination de durées comprend :
- un premier moyen monostable pour déterminer ladite première durée,
- un second moyen monostable pour déterminer ladite seconde durée, comprenant ledit moyen de compensation de sortie et ledit moyen de compensation d'entrée,
- des moyens logiques pour faire fonctionner alternativement lesdits premier et second moyens monostables pour former un oscillateur astable de période essentiellement égale à la somme desdites première et seconde durées.

Ledit second moyen monostable comprend :
- un condensateur pour déterminer une durée,
- un moyen pour remettre à l'état initial ledit condensateur à une première fraction préétablie de ladite tension de source d'entrée, quand lesdits moyens logiques ne font pas fonctionner ledit second moyen monostable,
- un moyen à seuil comprenant un moyen de compensation d'entrée pour déterminer le moment où la tension aux bornes dudit condensateur atteint une tension de seuil égale à une fraction préétablie de ladite tension de source d'entrée,
- un moyen de compensation de sortie pour charger ou décharger ledit condensateur vers ladite tension de seuil avec un courant essentiellement proportionnel à la tension de charge de sortie, et
- un moyen pour indiquer la fin dudit second intervalle auxdits moyens logiques, activé par ledit moyen à seuil.

Ladite première durée pour fermer ledit moyen de commutation est une valeur préétablie et fixe.

Ledit moyen de commutation est relié en série entre la borne positive de la tension de source d'entrée et l'inducteur.

Ledit moyen de commutation est constitué par un transistor à effet de champ en métal-oxyde-semiconducteur à canal p.

La figure 1 est une vue d'ensemble de l'invention appliquée à un défibrillateur implantable.

La figure 2 est un diagramme schématique du circuit de charge.

La figure 3 est un diagramme schématique du contrôleur.

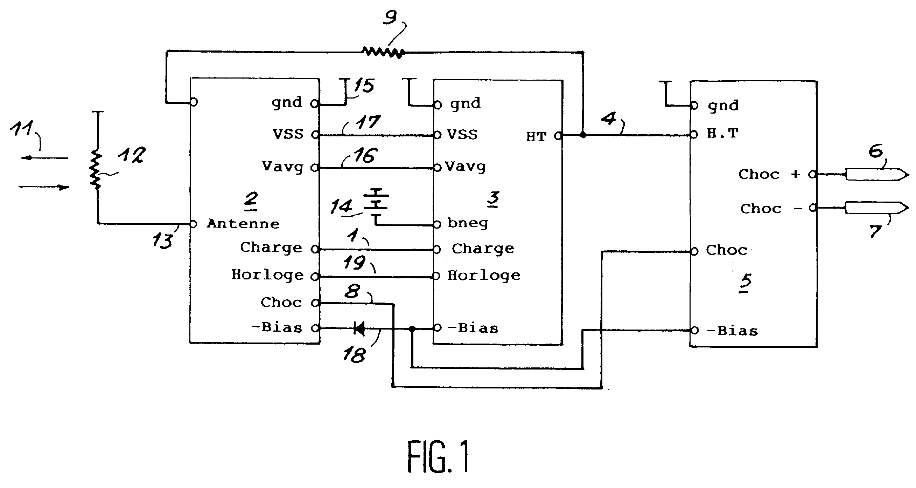

La figure 1 montre l'invention appliquée à un défibrillateur implantable. Lorsqu'il reçoit une commande de charge 1 par les circuits de basse puissance 2, le chargeur 3 fonctionne pour produire une haute tension 4, que les circuits de sortie de choc 5 appliquent à des bornes d'électrodes de choc 6 et 7 sur une commande de choc 8 provenant des circuits de basse puissance 2.

Les circuits de basse puissance 2 échantillonnent la haute tension 4 par l'intermédiaire de la résistance 9 pour déterminer le moment où la charge du chargeur atteint une tension cible. Les circuits de basse puissance assurent la commande de charge 1 chaque fois que le dispositif doit appliquer un choc et que cette tension de charge mesurée est inférieure à cette tension cible.

Les circuits de basse puissance 2 communiquent avec un dispositif externe par l'intermédiaire d'un système de communication électromagnétique 11 comprenant une antenne 12 et un port d'entrée-sortie 13.

La pile 14 alimente le dispositif en tension négative d'entrée bneg. La masse gnd en 15 sert de tension de référence (0 volt) pour tous les signaux du dispositif, sauf indication contraire. Le circuit chargeur 3 convertit la tension de la pile en d'autres tensions pour alimenter le reste du dispositif:
* haute tension HT en 4 pour le générateur 5 de choc, approximativement 0,75 KV. Le dispositif la rapporte à une alimentation de polarisation négative -bias 18 et non à la masse.
* tension moyenne de pile vavg en 16 pour faire fonctionner le chargeur 3 et fournir la puissance aux circuits de basse puissance 2. Pendant le fonctionnement du chargeur, la résistance interne de la pile, ainsi que les courants de pic élevés tirés pendant la phase d'entrée de l'inducteur, amènent la tension aux bornes de la pile à comporter des composantes de très haute fréquence. L'établissement de la moyenne de ladite tension de pile permet d'en réduire les fluctuations et de préserver un minimum de tension pour l'alimention générale des autres circuits de l'appareil.

Les circuits de basse puissance 2 régulent vavg 16 pour produire une alimentation logique vss en 17, d'approximativement -3,0 V, puis triplent vss pour produire une alimentation de polarisation négative -bias en 18, d'approximativement -9,0 V, qui est la tension la plus négative du dispositif. Quand le chargeur fonctionne, il amène la polarisation négative -bias à une tension encore plus négative ainsi que cela sera montré plus loin.

En se référant toujours à la figure 1, les circuits de basse puissance, en 2, procurent la stimulation, et la détection d'une manière classique, et commandent le chargeur 3 et le générateur de choc 5. Ils fonctionnent entre la masse 15 et la polarisation négative -bias en 18. Comme la valeur de la tension de stimulation est comprise entre ces deux tensions d'alimentation, ces circuits de basse puissance peuvent alors générer et mesurer les signaux de stimulation et de détection directement sans décalage de niveau. Comme le chargeur 3 fonctionne entre la masse et une tension d'entrée négative bneg, les circuits de basse puissance peuvent également commander le chargeur sans alimentation additionnelle en tension inversée. Si, au contraire, le chargeur fonctionnait entre la masse et une tension positive, le dispositif devrait inverser la tension de la pile pour produire la tension de stimulation.

Finalement, à la figure 1, les circuits de basse puissance procurent un signal d'horloge 19 au chargeur, typiquement à une fréquence qui est sensiblement inférieure à la fréquence de fonctionnement du système de communication en 11.

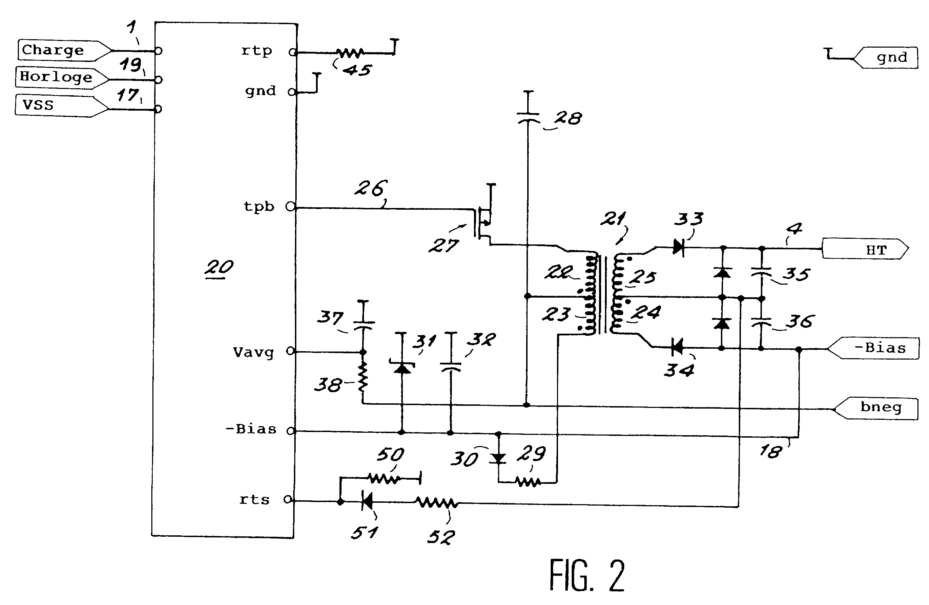

En se référant maintenant à la figure 2, le chargeur montré en 3 à la figure 1 consiste en un contrôleur 20 et des composants discrets.

Dans son mode de réalisation préféré, le chargeur comprend un transformateur 21, enroulé de préférence sur un noyau en pot RM5 réalisé en ferrite N48 de Siemens, avec A1 de 63 nH. L'enroulement primaire 22 est de préférence constitué par 13 spires, l'enroulement d'amplification 23 par 49 spires, et les enroulements haute tension 24 et 25 par 80 spires chacun.

Quand le contrôleur 20 valide le signal tpb en 26 à l'état bas, ceci fait démarrer la phase d'entrée. Le signal tpb rend le transistor 27 passant, appliquant la tension de la pile depuis la masse à bneg en passant par l'enroulement primaire 22, ce qui fait que le courant monte dans cet enroulement. Le condensateur 28 réduit les ondulations de la pile dues à l'impédance interne de cette pile. Pendant la phase d'entrée également, l'enroulement 23 multiplie la tension de la pile, et la résistance 29 et la diode 30 chargent en outre l'alimentation à polarisation négative -bias, limitée approximativement à -15V par la diode de Zener 31, et filtrée par le condensateur 32. Pendant la phase d'entrée, les diodes 33 et 34 sont polarisées en inverse, ainsi aucune énergie n'est transférée aux condensateurs de stockage 35 et 36.

Quand le contrôleur 20 rend valide le signal tpb en 26 à l'état haut, ceci commence la phase de sortie. Le signal tpb bloque le transistor 27, interrompant le courant passant dans l'enroulement primaire 22. Le courant passe alors dans les enroulements secondaires 24 et 25, chargeant les condensateurs de charge 35 et 36 par l'intermédiaire des diodes 33, 34. La tension aux bornes des enroulements 24 et 25 monte jusqu'à ce que les diodes 33 et 34 la limitent à la tension stockée dans les condensateurs 35 et 36 respectivement.

La pente de la rampe montante du courant pendant la phase d'entrée est égale à la tension de la pile Vp divisée par l'inductance Lp de l'enroulement 22. Ainsi, le courant de pic Ip dans l'enroulement 22 est Ip = VP*Tp / Lp, où Tp représente la durée de la phase d'entrée. Le rapport entre le courant de pic Is dans les enroulements 24 et 25 et le courant de pic Ip dans l'enroulement 22 est égal au nombre de spires Np de l'enroulement 22 divisé par la somme Ns du nombre de spires des enroulements 24 et 25. La pente de la rampe descendante du courant pendant la phase de sortie est égale à la tension totale Vs sur les condensateurs 35 et 36, divisée par l'inductance totale Ls des enroulements 24 et 25 combinés. En rappelant que L = Al * N^2 pour tout enroulement, on a alors Ts = (Ns/Np) * (Vp/Vs) * Tp, où Ts représente la durée nécessaire pour que l'énergie dans l'inducteur tombe à zéro pendant la phase de sortie. Ainsi, pour une durée fixe de la phase d'entrée Tp, Ts est directement proportionnel à la tension Vp de la source d'entrée et inversement proportionnel à la tension Vs de charge de sortie.

Toujours à la figure 2, le condensateur 37 et la résistance 38 produisent l'alimentation moyenne vavg à partir de la tension de la pile.

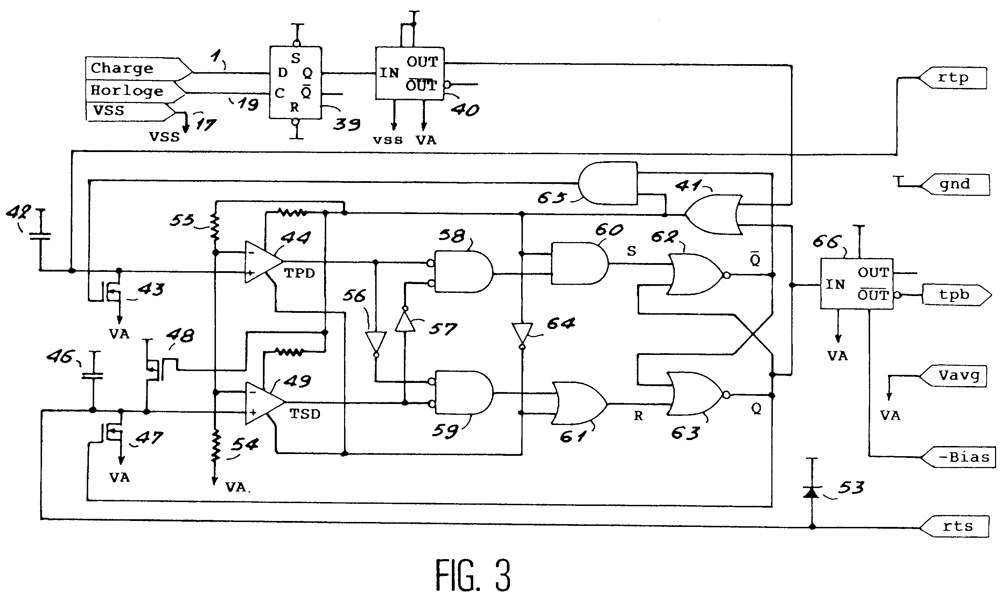

La figure 3 montre un circuit de contrôle détaillé. Les signaux 1 et 19 de commande du contrôleur et le verrou 39 fonctionnent entre la masse et vss en 17, car faire fonctionner autant de circuits que possible sur une basse tension réduit la consommation de puissance. Le signal de sortie tpb du contrôleur en 26 fonctionne entre la masse et la polarisation négative -bias, car la commande du commutateur 27 de la figure 2 à tension gâchette-source élevée réduit la résistance de commutation. Le reste du circuit du contrôleur de la figure 3 fonctionne entre la masse et vavg (symbolisé par VA) pour deux raisons. Tout d'abord, ce circuit fonctionne à haute fréquence et consomme donc un courant relativement élevé. Le faire fonctionner à partir de vavg au lieu de vss, réduit le besoin de charge sur le régulateur de vss. En second lieu, les composants analogiques du contrôleur fonctionnent sur une plage allant de vavg à vdd pour les raisons expliquées ci-dessous. Il est alors avantageux que les tensions de commande et de mesure du circuit sur ces composants fonctionnent également sur cette plage.

La logique du contrôleur est mise en oeuvre de préférence au moyen d'une technologie à métal-oxyde-semiconducteur à symétrie complémentaire (CMOS) de manière qu'elle ne tire pas de courant quand elle n'est pas validée. En outre, le circuit élimine le courant de polarisation des comparateurs 44 et 49 et du diviseur à résistances 54 et 55 quand il n'est pas validé. Il permet également d'être certain qu'il n'y a pas de courant de charge appliqué aux condensateurs 42 et 46 quand il n'est pas validé.

A la figure 3, le contrôleur comprend un circuit pour autoriser sélectivement le fonctionnement du chargeur, consistant en un verrou 39, un décaleur de niveau 40 et une porte 41. Le verrou 39 peut seulement changer d'état à la fréquence d'horloge 19 ou à une fréquence inférieure, quelle que soit la largeur du signal de demande de charge en 1. Le décaleur de niveau 40 adapte le signal de niveau vss à la sortie du verrou 39 à un signal de niveau vavg pour faire fonctionner la logique du contrôleur. Quand la sortie de 40 est à un niveau haut, ceci autorise le fonctionnement du convertisseur.

Le contrôleur comprend également des moyens logiques pour actionner des premier et second moyens monostables alternativement et de façon répétée, pour former un oscillateur astable, cette logique étant constituée par les portes 56 à 65 à la figure 3. Cette logique comprend un verrou de déclenchement/remise à zéro qui consiste en les portes 62 et 63. Quand la sortie Q du verrou, à la sortie de la porte 63, est à un niveau haut, elle ferme le commutateur de courant primaire 27 de la figure 2 par le décaleur de niveau 66. Ainsi, une sortie Q de niveau haut correspond à la phase d'entrée du convertisseur, et une sortie Q-barre de niveau haut à la sortie de la porte 62 correspond à la phase de sortie du convertisseur. Le décaleur de niveau 66 autorise le fonctionnement du commutateur de commande 27 à partir de l'alimentation en polarisation négative -bias.

La sortie de la porte 41 autorise un fonctionnement astable. La porte 41 assure, si la sortie 40 est à un niveau bas, l'arrêt du fonctionnement astable, alors que le convertisseur est dans la phase d'entrée, puis le convertisseur complète la phase d'entrée en cours avant de l'arrêter. Ceci permet d'être certain que chaque fois que le convertisseur effectue un cycle, il fournit une quantité maximale d'énergie aux condensateurs de charge, ce qui améliore le rendement du convertisseur.

La porte 60 empêche tout signal de déclencher le verrou (ce qui empêche la phase d'entrée), à moins que la porte 41 n'ait autorisé le fonctionnement astable. De façon similaire, la porte 61 et l'inverseur 64 remettent le verrou à l'état initial (ce qui force la phase de sortie), à moins que la porte 41 n'ait autorisé le fonctionnement astable.

Par l'intermédiaire de l'inverseur 57 et de la porte 58, un niveau haut en TSD, en sortie du comparateur 49, déclenche le verrou (mettant fin à la phase de sortie) à condition qu'il y ait un niveau bas en TPD, en sortie du comparateur 44. De façon similaire, par l'intermédiaire de l'inverseur 56 et de la porte 59, un niveau haut en TPD remet à l'état initial le verrou (mettant fin à la phase d'entrée) à condition qu'il y ait un niveau bas en TSD. Les portes 58 et 59 bloquent tout déclenchement ou remise à l'état initial quand il y a un niveau haut à la fois sur TPD et sur TSD.

Finalement, pour terminer la description des moyens logiques, la porte 65 fournit un niveau haut quand un fonctionnement astable est autorisé et quand le verrou est remis à l'état initial (phase de sortie).

Le contrôleur comprend un moyen de seuil consistant en un diviseur résistif 54, 55 qui définit le seuil du comparateur pour deux circuits monostables discutés plus bas, à une fraction préétablie de la tension moyenne de la pile, quand le fonctionnement du chargeur est autorisé.

Le contrôleur comprend également un premier moyen monostable pour déterminer la durée de la phase d'entrée, consistant en un condensateur 42, un commutateur de remise à l'état initial 43, et un comparateur 44, tous montrés à la figure 3, et en la résistance 45 de la figure 2. En supposant qu'un fonctionnement astable soit autorisé : le commutateur 43 maintient le condensateur 42 chargé sur vavg pendant la phase de sortie. Au début de la phase d'entrée, la résistance 45 décharge le condensateur 42 vers la masse. Quand la borne négative du condensateur 42 atteint la tension de seuil définie par les résistances 54 et 55, la sortie TPD du comparateur passe à un état haut, remettant à l'état initial le verrou 62, 63 comme expliqué ci-dessus, mettant ainsi fin à la phase d'entrée et faisant démarrer la phase de sortie. Pendant la phase d'entrée, le condensateur 42 se décharge jusqu'à une fraction fixée de sa tension initiale par l'intermédiaire d'une résistance fixe; ainsi, sa période est indépendante de cette tension initiale.

Le contrôleur comprend en outre un second moyen monostable pour déterminer la durée de la phase de sortie, consistant en un condensateur 46, des commutateurs de remise à l'état initial 47 et 48, une diode 53 et un comparateur 49, qui sont tous montrés à la figure 3; et une résistance 50, une diode 51 et une résistance 52 qui sont montrées à la figure 2. En supposant que le fonctionnement astable soit autorisé: le commutateur 47 maintient le condensateur 46 chargé à vavg pendant la phase d'entrée. Au début de la phase de sortie, la résistance 50 décharge le condensateur 46 vers la masse, et la résistance 52 qui est en série avec la diode 51 décharge également le condensateur 46 vers la masse. Quand la borne négative du condensateur 46 atteint la tension de seuil établie par les résistances 54 et 55, la sortie TSD du comparateur 49 passe à l'état haut, positionnant le verrou 62, 63 comme expliqué ci-dessus, mettant ainsi fin à la phase de sortie et faisant démarrer la phase d'entrée.

En l'absence de tout courant passant par la résistance 52, le condensateur 46 se décharge jusqu'à une fraction fixe de sa tension initiale par l'intermédiaire d'une résistance fixe 50, et sa période est donc indépendante de celle de la tension initiale. Dans la pratique, le condensateur 46 et la résistance 50 sont sélectionnés de manière que la fréquence astable du convertisseur soit toujours sensiblement plus élevée que la fréquence de fonctionnement du système de communication 11 qui est d'approximativement 8 KHz dans cet exemple. Tout courant passant par la résistance 52 fait seulement accélérer le convertisseur.

Quand les condensateurs de charge 35 et 36 sont chargés jusqu'à quelques dizaines de volts, la résistance 52 applique le courant de décharge au condensateur 46, ce courant étant essentiellement directement proportionnel à la tension du condensateur de sortie à la jonction entre les condensateurs de stockage d'énergie 35 et 36, et de façon significative supérieur au courant passant par la résistance 50, ce qui fait que la résistance 52 à elle seule détermine essentiellement la durée de la phase de sortie déterminée par ce circuit. Comme la tension de seuil à la jonction des résistances 54 et 55 est une fraction de la tension de la pile, et comme la pente de la décharge du condensateur 46 est proportionnelle à la tension de charge de sortie, la durée de la phase de sortie est alors directement proportionnelle à la tension de la pile et inversement proportionnelle à la tension de sortie.

| **Tableau montrant des valeurs typiques des composants** | | | |
|---|---|---|---|
| **Composant** | **type** | **valeur** | **notes** |
| 9 | résistance | 39 M | 1 KV |
| | | | |
| 14 | pile | 6,4 V | bas esr |
| | | | |
| 27 | MOSFET à canal p | 0,3 Ohm, 60V | IRFU9024 |
| | | | |
| 28 | condensateur tantale | 10 µF,10V | bas esr |
| | | | |
| 29 | résistance | 0,68K | |
| | | | |
| 30 | diode à récup. ultra-rapide | 400PIV, 1,0A | BYV26C |
| | | | |
| 31 | diode Zener | 15V | |
| | | | |
| 32 | condensateur tantale | 1,0µF, 50V | |
| | | | |
| 33,34 | diode à récup. ultra-rapide | 600PIV,1,0 A | BYV26E |
| | | | |
| 35, 36 | cond. électrolytique Aluminium | 250 µF,360 V | |
| | | | |
| 37 | condensateur tantale | 10 µF,10V | |
| | | | |
| 38 | résistance | 33 Ohms | |
| | | | |
| 42, 46 | condensateur intégré | 40 pF | |
| | | | |
| 45 | résistance, sélection sur test, établit durée phase d'entrée | 0,20 M à 0,50 M | |
| | | | |
| 50 | résistance | 1,0 M | |
| | | | |
| 51, 53 | petite diode de signalisation | 20 PIV, 1,0 A | 1N4148 |
| | | | |
| 52 | résistance | 2,5 M | 500 V |

## Revendications

1. Convertisseur continu-continu, comprenant :
- un moyen inductif (22) pour stocker l'énergie,
- un moyen de commutation (27) pour autoriser sélectivement le courant d'entrée de l'inducteur,
- un moyen à diode (33, 34) pour autoriser le courant de sortie de l'inducteur seulement quand le courant d'entrée de l'inducteur est sensiblement nul,
- un moyen de contrôle (20) pour faire effectuer de façon répétée des cycles audit moyen de commutation (27) pour qu'il se ferme pendant une première durée de conduction (P), puis qu'il s'ouvre pendant une seconde durée de blocage (Ts) et,
- un moyen de compensation de sortie (52),
caractérisé en ce que le le moyen de compensation de sortie (52) ajuste ladite seconde durée (Ts) en proportion inverse de la tension de charge de sortie Vs).

2. Convertisseur selon la revendication 1, caractérisé en ce que la tension de charge de sortie (Vs) est la tension moyenne de charge de sortie.

3. Convertisseur selon la revendication 1, caractérisé en ce que la tension de charge de sortie (Vs) est la tension de pic de charge de sortie.

4. Convertisseur selon l'une quelconque des revendications 1 à 3, caractérisé en ce que ledit moyen de contrôle (20) comprend :
- un premier moyen monostable (42-45) pour déterminer ladite première durée (Tp),
- un second moyen monostable (46-53) pour déterminer ladite seconde durée (Ts), comprenant ledit moyen de compensation de sortie (52),
- un moyen logique (56-65) pour faire fonctionner de façon alternée et répétée lesdits premier et second moyens monostables pour former un oscillateur astable dont la période est essentiellement égale à la somme desdites première (Tp) et seconde (Ts) durées.

5. Convertisseur selon la revendication 4, caractérisé en ce que ledit second moyen monostable (46-53) comprend :
- un condensateur (46) pour déterminer une durée,
- un moyen (47) pour remettre à l'état initial ledit condensateur à une première tension prédéterminée lorsque lesdits moyens logiques (56-65) ne font pas fonctionner ledit second moyen monostable,
- un moyen à seuil (54, 55) pour déterminer le moment où la tension aux bornes dudit condensateur (46) atteint une tension de seuil prédéterminée,
- un moyen de compensation de sortie (52) pour charger ou décharger ledit condensateur vers ladite tension de seuil à un courant essentiellement proportionnel à la tension de charge de la sortie,
- un moyen (49) pour indiquer la fin dudit second intervalle auxdits moyens logiques (56-65), activé par ledit moyen à seuil (54, 55).

6. Convertisseur selon la revendication 5, caractérisé en ce que ledit moyen pour charger ou décharger comprend une résistance (52) branchée entre la tension de charge de sortie et la borne dudit condensateur reliée audit moyen à seuil.

7. Convertisseur selon l'une quelconque des revendications 1 à 6, caractérisé en ce qu'il comprend un moyen de compensation d'entrée additionnel pour ajuster ladite seconde durée en proportion directe de la tension de source d'entrée.

8. Convertisseur selon la revendication 7, caractérisé en ce que ladite tension de source d'entrée est la tension moyenne de source d'entrée.

9. Convertisseur selon la revendication 7, caractérisé en ce que ladite tension de source d'entrée est la tension de pic de source d'entrée.

10. Convertisseur selon l'une quelconque des revendications 7 à 9, caractérisé en ce que ledit moyen de détermination de durées comprend :
- un premier moyen monostable pour déterminer ladite première durée,
- un second moyen monostable pour déterminer ladite seconde durée, comprenant ledit moyen de compensation de sortie et ledit moyen de compensation d'entrée,
- des moyens logiques pour faire fonctionner alternativement lesdits premier et second moyens monostables pour former un oscillateur astable de période essentiellement égale à la somme desdites première et seconde durées.

11. Convertisseur selon la revendication 10, caractérisé en ce que ledit second moyen monostable comprend :
- un condensateur pour déterminer une durée,
- un moyen pour remettre à l'état initial ledit condensateur à une première fraction préétablie de ladite tension de source d'entrée, quand lesdits moyens logiques ne font pas fonctionner ledit second moyen monostable,
- un moyen à seuil comprenant un moyen de compensation d'entrée pour déterminer le moment où la tension aux bornes dudit condensateur atteint une tension de seuil égale à une fraction préétablie de ladite tension de source d'entrée,
- un moyen de compensation de sortie pour charger ou décharger ledit condensateur vers ladite tension de seuil avec un courant essentiellement proportionnel à la tension de charge de sortie, et
- un moyen pour indiquer la fin dudit second intervalle auxdits moyens logiques, activé par ledit moyen à seuil.

12. Convertisseur selon la revendication 11, caractérisé en ce que ledit moyen pour charger ou décharger comprend une résistance branchée entre la tension de charge secondaire et la borne du condensateur reliée audit moyen à seuil.

13. Convertisseur selon l'une quelconque des revendications 1 à 12, caractérisé en ce que ladite première durée pour fermer ledit moyen de commutation est une valeur préétablie et fixe.

14. Convertisseur selon l'une quelconque des revendications 1 à 13, caractérisé en ce que ledit moyen de commutation est relié en série entre la borne positive de la tension de source d'entrée et l'inducteur.

15. Convertisseur selon la revendication 14, caractérisé en ce que ledit moyen de commutation est constitué par un transistor à effet de champ en métal-oxyde-semiconducteur à canal p.

## Patentansprüche

1. Gleichstrom-Gleichstrom-Umformer mit:
- einer induktiven Einrichtung (22) zur Energiespeicherung,
- einer Schalteinrichtung (27), um den Eingangsstrom der induktiven Einrichtung wahlweise zuzuführen,
- einer Diodeneinrichtung (33, 34) zur ausschließlichen Freigabe des Ausgangsstroms der induktiven Einrichtung, wenn der Eingangsstrom der induktiven Einrichtung ungefähr Null beträgt,
- einer Steuerungseinrichtung (20) zum wiederholten Bewirken von Zyklen der Schalteinrichtung (27), damit diese während einer ersten Dauer des Leitens (P) geschlossen ist, und dann während einer zweiten Dauer des Sperrens (Ts) geöffnet ist, und
- einer Ausgangs-Abgleicheinrichtung (52),
**dadurch gekennzeichnet, daß**
die Ausgangs-Abgleicheinrichtung (52) die zweite Dauer (Ts) im umgekehrten Verhältnis zur Ausgangs-Ladespannung (Vs) einstellt.

2. Umformer nach Anspruch 1,
**dadurch gekennzeichnet, daß**
die Ausgangs-Ladespannung (Vs) die Mittelwert-Ausgangs-Ladespannung ist.

3. Umformer nach Anspruch 1,
**dadurch gekennzeichnet, daß**
die Ausgangs-Ladespannung (Vs) die Spitzenwert-Ausgangs-Ladespannung ist.

4. Umformer nach einem der Ansprüche 1 bis 3,
**dadurch gekennzeichnet, daß**
die Steuerungseinrichtung (20)
- eine erste monostabile Einrichtung (42-45) zur Bestimmung der ersten Dauer (Tp),
- eine zweite monostabile Einrichtung (46-53) zur Bestimmung der zweiten Dauer (Ts), welche die Ausgangs-Abgleicheinrichtung (52) enthält, und
- Logikeinrichtungen (56-65) zum alternierenden und wiederholten Betreiben der ersten und zweiten monostabilen Einrichtungen, um einen astabilen Oszillator auszubilden, dessen Periode im wesentlichen gleich der Summe der ersten (Tp) und zweiten (Ts) Dauer ist, umfaßt.

5. Umformer nach Anspruch 4, **dadurch gekennzeichnet, daß** die zweite monostabile Einrichtung (46-53)
- einen Kondensator (46) zur Bestimmung einer Dauer,
- eine Einrichtung (47) zum Rücksetzen des Kondensators in den Ausgangszustand auf eine erste vorbestimmte Spannung, wenn die Logikeinrichtungen (56-65) die zweite monostabile Einrichtung nicht betreiben,
- eine Schwelleneinrichtung (54, 55) zur Bestimmung des Zeitpunkts, zu dem die Spannung an den Anschlüssen des Kondensators (46) eine vorbestimmte Schwellenspannung erreicht,
- eine Ausgangs-Abgleicheinrichtung (52) zum Laden oder Entladen des Kondensators in Richtung der Schwellenspannung mit einem zur Ausgangs-Ladespannung im wesentlichen proportionalen Strom, und
- eine Einrichtung (49), die den Logikeinrichtungen (56-65) das Ende des zweiten Intervalls anzeigt und mittels der Schwelleneinrichtung (54, 55) aktiviert wird, umfaßt.

6. Umformer nach Anspruch 5,
**dadurch gekennzeichnet, daß**
die Einrichtung zum Laden oder Entladen einen Widerstand (52) aufweist, der zwischen die Ausgangs-Ladespannung und den an die Schwelleneinrichtung angeschlossenen Anschluß des Kondensators geschaltet ist.

7. Umformer nach einem der vorhergehenden Ansprüche 1 bis 6, **dadurch gekennzeichnet, daß**
er eine zusätzliche Eingangs-Abgleicheinrichtung zur Einstellung der zweiten Dauer in direktem Verhältnis zur Eingangsquellenspannung aufweist.

8. Umformer nach Anspruch 7,
**dadurch gekennzeichnet, daß**
die Eingangsquellenspannung die Mittelwert-Eingangsquellenspannung ist.

9. Umformer nach Anspruch 7,
**dadurch gekennzeichnet, daß**
die Eingangsquellenspannung die Spitzenwert-Eingangsquellenspannung ist.

10. Umformer nach einem der vorhergehenden Ansprüche 7 bis 9, **dadurch gekennzeichnet, daß**
die Einrichtung zur Bestimmung der Dauer
- eine erste monostabile Einrichtung zur Bestimmung der ersten Dauer,
- eine zweite monostabile Einrichtung zur Bestimmung der zweiten Dauer, welche die Ausgangs-Abgleicheinrichtung und die Eingangs-Abgleicheinrichtung umfaßt, und
- Logikeinrichtungen zum alternierenden Betreiben der ersten und zweiten monostabilen Einrichtungen, um einen astabilen Oszillator auszubilden, der eine Periode hat, die im wesentlichen gleich der Summe der ersten und zweiten Dauer ist, umfaßt.

11. Umformer nach Anspruch 10,
**dadurch gekennzeichnet, daß**
die zweite monostabile Einrichtung
- einen Kondensator zur Bestimmung einer Dauer,
- eine Einrichtung zum Rücksetzen des Kondensators in den Ausgangszustand auf einen ersten voreingestellten Bruchteil der Eingangsquellenspannung, wenn die Logikeinrichtungen die zweite monostabile Einrichtung nicht betreiben,
- eine Schwelleneinrichtung mit einer Eingangs-Abgleicheinrichtung zur Bestimmung des Zeitpunktes, zu dem die Spannung an den Anschlüssen des Kondensators eine Schwellenspannung erreicht, die gleich einem voreingestellten Bruchteil der Eingangsquellenspannung ist,
- eine Ausgangs-Abgleicheinrichtung zum Laden oder Entladen des Kondensators in Richtung der Schwellenspannung mit einem Strom, der im wesentlichen proportional zur Ausgangs-Ladespannung ist, und
- eine Einrichtung, die den Logikeinrichtungen das Ende des zweiten Intervalls anzeigt und mittels der Schwelleneinrichtung aktiviert wird, umfaßt.

12. Umformer nach Anspruch 11,
**dadurch gekennzeichnet, daß**
die Einrichtung zum Laden oder Entladen einen Widerstand aufweist, der zwischen die Sekundär-Ladespannung und den an die Schwelleneinrichtung angeschlossenen Anschluß des Kondensators geschaltet ist.

13. Umformer nach einem der vorhergehenden Ansprüche 1 bis 12, **dadurch gekennzeichnet, daß**
die erste Dauer zum Schließen der Schalteinrichtung einen voreingestellten und festen Wert hat.

14. Umformer nach einem der vorhergehenden Ansprüche 1 bis 13, **dadurch gekennzeichnet, daß**
die Schalteinrichtung in Reihe zwischen den positiven Anschluß der Eingangsquellenspannung und die induktive Einrichtung geschaltet ist.

15. Umformer nach Anspruch 14,
**dadurch gekennzeichnet, daß**
die Schalteinrichtung aus einem P-Kanal-Feldeffekttransistor der Metall-Oxid-Halbleiter-Bauart besteht.

## Claims

1. DC/DC converter including :
- means (22) for inductively storing energy,
- switch means (27) for selectively enabling the inductor input current;
- diode means (33,34) for enabling the inductor output current only when the inductor input current is substantially zero;
- control means (20) for repeatedly cycling the switch means (27) to close for a first conduction duration (P) and then to open for a second blocking duration (Ts); and
- output compensating means (52),
characterized in that said output compensating means (52) adjusts said second duration (Ts) in inverse proportion to the output load voltage (Vs).

2. Converter according to claim 1 characterized in that the output load voltage (Vs) is the average output load voltage.

3. Converter according to claim 1 characterized in that the output load voltage (Vs) is the output load peak voltage.

4. Converter according to anyone of claims 1 to 3, characterized in that said control means (20) includes :
- a first monostable means (42-45) for timing said first duration (Tp),
- a second monostable means (46-53) for timing said second duration (Ts), including said output compensating means (52),
- a logic means (56-65) for alternately and repeatedly operating said first and second monostable means to form an astable oscillator with a period essentially equal to the sum of said first (Tp) and second (Ts) durations.

5. Converter according to claim 4, characterized in that said second monostable means (46-53) includes :
- a capacitor (46) for timing a duration,
- means for resetting said capacitor to a first preset voltage when said logic means (56-65) do not operate said second monostable means,
- threshold means (54-55) for determining when the voltage across said capacitor (46) reaches a preset threshold voltage,
- output compensating means (52) for charging or discharging said capacitor toward said threshold voltage at a current essentially proportional to the output load voltage, and
- means (49) for indicating completion of said second duration to said logic means (56-65), activated by said threshold means (54,55).

6. Converter according to claim 5, characterized in that said means for charging or discharging includes a resistor (52) connected between the output load voltage and the terminal of said capacitor connected to said threshold means.

7. Converter according to anyone of claims 1 to 6, characterized in that it includes an additional input compensating means for adjusting said second duration in direct proportion to the input source voltage.

8. Converter according to claim 7, characterized in that said input source voltage is the average input source voltage.

9. Converter according to claim 7, characterized in that said input source voltage is the input source peak voltage.

10. Converter according to anyone of claims 7 to 9, characterized in that said control means includes :
- first monostable means for timing said first duration,
- second monostable means for timing said second duration, including said output compensating means and said input compensating means, and
- logic means for alternately operating said first and second monostable means to form an astable oscillator with a period essentially equal to the sum of said first and second durations.

11. Converter according to claim 10, characterized in that said second monostable means includes :
- a capacitor for timing a duration,
- means for resetting said capacitor to a first preset fraction of said input source voltage, when said logic means do not operate said second monostable means,
- threshold means including an input compensating means for determining when the voltage across said capacitor reaches a threshold voltage equal to a preset fraction of said input source voltage,
- output compensating means for charging or discharging said capacitor toward said threshold voltage with a current essentially proportional to output load voltage, and
- means for indicating completion of said second interval to said logic means, which are activated by said threshold means.

12. Converter according to claim 11, characterized in that said means for charging or discharging includes a resistor connected between secondary load voltage and the capacitor terminal connected to said threshold means.

13. Converter according to anyone of claims 1 to 12, characterized in that said first duration for closing said switch means is a fixed preset value.

14. Converter according to anyone of claims 1 to 13, characterized in that said switch means is connected in series between the positive terminal of the input source voltage and the inductor.

15. Converter according to claim 14, characterized in that said switch means is a p-channel metal-oxide-semiconductor field-effect-transistor (MOSFET).
